# EUROPEAN PATENT APPLICATION

(11) **EP 1 770 570 A2**
(43) Date of publication of application: **04.04.2007**
(21) Application number: 06120852.6
(22) Date of filing: 18.09.2006
(51) Int. Cl.: G06F 19/00

(54) **System and method for identification of images in an image database**

(30) Priority: 18.09.2005 IL 17093705; 19.09.2005 US 717774 P
(71) Applicant: Given Imaging Ltd., 20692 Yoqneam (IL)
(72) Inventor: HORN, Eli, 26315, Kiryat Motzkin (IL); MERON, Gavriel, 49556, Petach Tikva (IL); DAVIDSON, Tal, 20692, Yoqneam Illit (IL)
(74) Representative: Harrison Goddard Foote

(57) **Abstract**

Embodiments of the present invention provide a system and method for searching for a specific image or another feature in a database, for example, a database of pathologies, the image being produced by for example an in vivo imaging device such as an ingestible capsule.

## Description

### FIELD OF THE INVENTION

The present invention relates to identification of an image or part of an image for example using a database of pathologies or other data.

### BACKGROUND OF THE INVENTION

When viewing a moving image, for example which may be used for medical diagnosis, the viewer, may desire to record comments regarding certain portions or frames, or may wish to "bookmark" certain portions or frames.

For example, an in vivo imager system carried by an ingestible capsule may be used to image lumens within a patient. The imager system captures and transmits, for example, images of the gastrointestinal (GI) tract to an external recording device while the capsule passes through the GI lumen. Such an in vivo imaging system provides a platform from which moving or still images of a lumen may be viewed. Large numbers of images may be collected for viewing. For example, the images may be combined in sequence, and a moving image of, for example, 40 minutes in length, may be presented to the user. It would be desirable to enable a user to note significant details or portions of such a set of images.

### SUMMARY OF THE INVENTION

An exemplary embodiment of the present invention provides a system and method for searching for a specific image or another feature in a database, for example, a database of pathologies, the image being produced by, for example, an in vivo imaging device such as an ingestible capsule. A workstation or other device may accept acquired images and may display the images on a monitor A health professional may, for example, choose or identify a specific image captured by the in vivo device and the system may search the database for a matching image another reference item in order to, for example, confirm a diagnosis or get additional information.

Another embodiment of the present invention provides a system and method for identifying pathalogy in the GI tract, the method may include displaying in vivo images of the GI tract obtained by an in-vivo imaging device, accepting an indication of an image of interest from the in vivo images and searching for an item from a database, the item matching the image of interest.. According to some embodiments of the present invention the searching may be preformed by entering a word relating to the image of interest. According to some embodiments of the present invention the search results and the image of interest may be displayed simultaneously.

Another embodiment of the present invention provides a system and method for reviewing in vivo images, the system may include an in-vivo imaging device to collect in-vivo images, a pathology database, a processor to match an in vivo image with an item in the pathology database and a display to display the in-vivo images.

### BRIEF DESCRIPTION OF THE DRAWINGS

The present invention will be understood and appreciated more fully from the following detailed description taken in conjunction with the drawings in which:
Fig. 1 shows a schematic diagram of an imaging system according to one embodiment of the present invention;
Fig. 2 depicts a flowchart of an image identification method, according to one embodiment of the present invention;
Fig. 3 depicts a flowchart of an image comparison method, according to one embodiment of the present invention; and
Fig. 4 shows a display of a monitor according to one embodiment of the present invention.

### DETAILED DESCRIPTION OF THE INVENTION

In the following description, various aspects of the present invention will be described. For purposes of explanation, specific configurations and details are set forth in order to provide a thorough understanding of the present invention. However, it will also be apparent to one skilled in the art that the present invention may be practiced without the specific details presented herein. Furthermore, well-known features may be omitted or simplified in order not to obscure the present invention.

Unless specifically stated otherwise, as apparent from the following discussions, it is appreciated that throughout the specification discussions utilizing terms such as "processing", "computing", "storing", "determining", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. Such apparatuses may be specially constructed for the desired purposes, or may comprise general purpose computers selectively activated or reconfigured by a computer program stored in the computers. Such computer programs may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes and displays presented herein are not inherently related to any particular computer or other apparatus. Various general purpose systems may be used with programs in accordance with the teachings herein, or it may prove convenient to construct a more specialized apparatus to perform the desired method. It will be appreciated that a variety of programming languages may be used to implement the teachings of the invention as described herein,

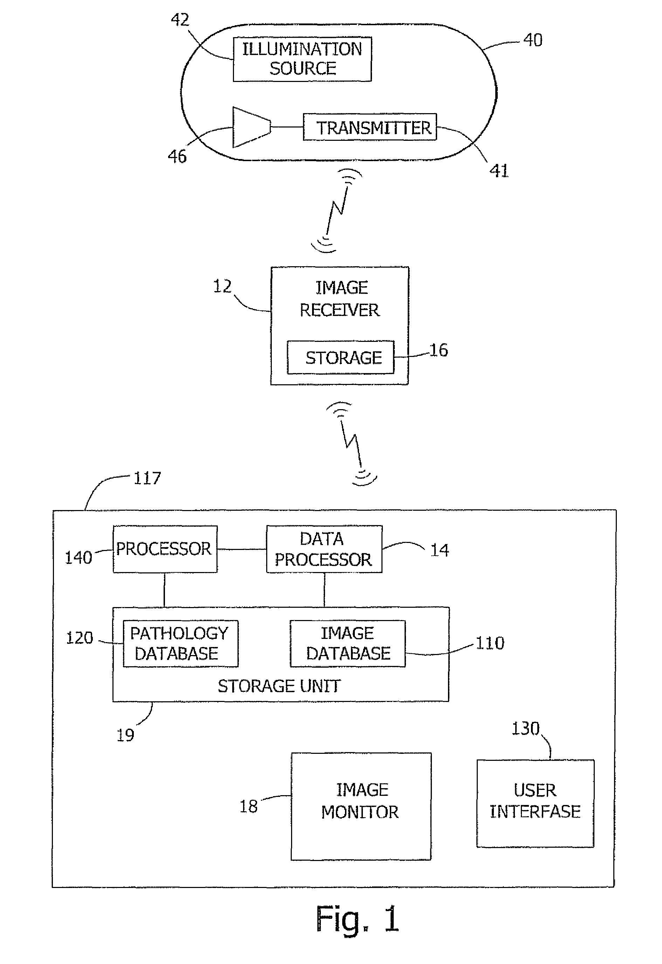

Reference is made to Fig. 1, which shows a schematic diagram of an imaging system according to one embodiment of the present invention. In an exemplary embodiment, the system includes an in vivo device 40, for example a capsule or other suitable device, having an imager 46, for capturing images, an illumination source 42, for illuminating the body lumen, and a transmitter 41, for transmitting image and possibly other information to a receiving device. In alternate embodiments device 40 may be other than a capsule; for example, device 40 may be an endoscope, an non-in vivo imaging device, etc. An optical system (not shown), including, for example, lenses or mirrors, may aid in focusing reflected light onto the imager 46. The device 40 is inserted into a patient for example by swallowing and typically traverses the patient's GI tract. In certain embodiments, the device and image capture system are similar to embodiments described in U.S. Patent No. 5,604,531 or in U.S. Application No. 09/800,470, both assigned to the common assignee of the present application and incorporated by reference herein. In alternate embodiments, other image capture devices, having other configurations, and other image capture systems, having other configurations, may be used.

Device 40 typically may be or may include an autonomous swallowable capsule, but device 40 may have other shapes and need not be swallowable or autonomous. Embodiments of device 40 are typically autonomous, and are typically self-contained. For example, device 40 may be a capsule or other unit
where all the components are substantially contained within a container or shell, and where device 40 does not require any wires or cables to, for example, receive power or transmit information. In some embodiments, device 40 may be autonomous and non-remote-controllable; in another embodiment, device 40 may be partially or entirely remote-controllable.

Preferably, located outside the patient's body in one or more locations, are an image receiver/recorder 12, preferably including an antenna or antenna array, an image receiver storage unit 16, a data processor 14, a processor 140, a data processor storage unit 19, an image monitor 18, for displaying, *inter alia,* the images recorded by the device 40 and other information, and a user interface 130.

Preferably, the image receiver/recorder 12 and image receiver storage unit 16 are small and portable, and can be worn on the patient's body during recording of the images. Data processor storage unit 19 may include an image database 110 and a pathology or other database 120. Preferably, data processor 14, processor 140 data processor storage unit 19 and monitor 18 are part of a personal computer or workstation , such as workstation 117 which may include standard components such as processor 14, a memory, a disk drive, and input-output devices, although alternate configurations are possible. The system and method of the present invention may be implemented on various suitable computing systems. Database 120 may be in other locations, and may store information other than pathologies, for example, database 120 may be remote or accessed via a network such as the Internet.

Data processor 14 may include any suitable data processor, such as a microprocessor, multiprocessor, accelerator board, or any other serial or parallel high performance data processor. Image monitor 18 may be a computer screen, a conventional video display, or any other device capable of providing image or other data.

Preferably, the imager 46 is a suitable CMOS camera such as a "camera on a chip" type CMOS imager. In alternate embodiments, the imager 46 may be another device, for example, a CCD. The illumination source 42 may be, for example, one or more light emitting diodes, or another suitable light source.

Transmitter 41 may operate using radio waves; but in some embodiments, such as those where device 40 is or is included within an endoscope, transmitter 41 may transmit/receive data via, for example, wire, optical fiber and/or other suitable methods, RF or other known wireless methods of transmission may be used Transmitter 41 may include, for example, a transmitter module or sub-unit and a receiver module or sub-unit, or an integrated transceiver or transmitter-receiver. Other configurations are possible.

In operation, imager 46 captures images and sends data representing the images to transmitter 41, which transmits images to image receiver/recorder 12 using, for example, electromagnetic radio waves. Image receiver/recorder 12 may transfer the image data to image receiver storage unit 16. After a certain time of data collection, the image data stored in storage unit 16 may be sent to the data processor 14 or the data processor storage unit 19. For example, the image receiver storage unit 16 may be taken off the patient's body and connected to a personal computer or workstation which includes the data processor 14 and data processor storage unit 19 via a standard data link, *e.g.,* a serial or parallel interface of known construction. The image data may then be transferred from the image receiver storage unit 16 to the image database 110, which may be within data processor storage unit 19. Data processor 14 may analyze the data and provide the analyzed data to the image monitor 18, where a health professional may view the image data. Data processor 14 may operate software (not shown) which, in conjunction with basic operating software such as an operating system and device drivers, may control the operation of data processor 14. Preferably, the software controlling data processor 14 includes code written in the C++ language and possibly additional languages, but may be implemented in a variety of known methods.

In other embodiments intermediate image receiver/recorder 12 need not be used.

Pathology or other database 120 which is typically included in storage unit 19 may be contained within for example a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storage. The pathology database 120 may contain different images of, for example, known pathologies or cases, known diseases, tumors, specific tissues or other reference images. Known pathologies may include polyps, lesions, bleeding etc. Pathology database 120 may also contain other information related to each image, for example, text information, keywords, descriptions, a complete medical diagnosis, relevant cases, articles or additional images, for example, images of close areas, images of the same pathology at different time points or any other information In other embodiments, other or additional reference data may be contained within database 120 for the purpose of being compared with images from an image stream.

The image data collected and stored may be stored indefinitely, transferred to other locations, or manipulated or analyzed. A health professional may use the images to diagnose pathological conditions of the GI tract, and, in addition, the system may provide information about the location of these pathologies. While, using a system where the data processor storage unit 19 first collects data and then transfers data to the data processor 14, the image data is typically not viewed in real time, other configurations allow for real time viewing.

The image monitor 18 presents the image data, preferably in the form of still and moving pictures, and in addition may present other information. In an exemplary embodiment, such additional information may include, but is not limited to, absolute time elapsed for the current image being shown and annotations or summary information for annotations. Absolute time elapsed for the current image being shown may be, for example:
a. the amount of time that elapsed between the moment the device 40 was first activated and the moment that the current image being displayed was captured, or
b. the amount of time that elapsed between the image receiver 12 started receiving transmission from the device 40 and the moment that the current image being displayed was captured. In other embodiments, time elapsed may include other periods, such as time elapsed from the start of a stream of images to the current point. In further embodiments measurements such as number of frames elapsed may be used. In an exemplary embodiment, various categories of information may be displayed in windows. In other embodiments multiple monitors may be used to display image and other data.

Preferably, the in vivo imager system collects a series of still images as it traverses the GI tract. The images may be later presented as, for example, a stream of images or a moving image of the traverse of the GI tract. The in vivo imager system may collect a large volume of data, as the in vivo device 40 may take several hours to traverse the GI tract, and may record images at a rate of, for example, two images every second, resulting in the recordation of thousands of images. The image recordation rate (or frame capture rate) may be varied.

Preferably, the image data recorded and transmitted by the device 40 is digital color image data, although in alternate embodiments, other image formats may be used. In an exemplary embodiment, each frame of image data includes 256 rows of 256 pixels each, each pixel including bytes for color and brightness, according to known methods. For example, in each pixel, color may be represented by a mosaic of four sub-pixels, each sub-pixel corresponding to primaries such as red, green, or blue (where one primary is represented twice). The brightness of the overall pixel may be recorded by a one byte (*e.g.* 0-255) brightness value. Preferably, images are stored sequentially in data processor storage unit 19. The stored data is comprised of one or more pixel properties, including color and brightness. Other data formats and image formats may be used.

A health professional may come across an image of interest when viewing the image data received from the in vivo device, may select or mark the images or a set (where set may include one or more item) of images (including, for example, a sub-stream of images) and may, for example, decide to search pathology database 120 for, for example, a matching image, a reference image, a similar image, a matching area of an image, a matching group of pixels or any other part of an image. A search may also be preformed for a match between an image in an image stream and non-image data in database 120; for example a match between an image and a text description, etc. In one embodiment of the invention the search may be performed based on a set of parameters (wherein set can include one or more items), for example, an image, part of an image and keywords. A search tool may be used to search database 120 for images that are highly correlated with the selected image, in addition to search text associated with the images according to specific keywords. In one embodiment a user may prioritize a search according to a specific parameter, for example, priority may be given to the keyword search. In another embodiment a search may be performed based on correlation to the selected image and another search may be done on the first search results based on another parameter, for example, a user may refine the search results by adding a keyword.

In one embodiment, a user may send a command via for example user interface 130, which may be, for example, a keyboard, a joystick, a mouse or any other suitable input-output device to data processor 14 in data processor storage unit 19. Data processor 14 may operate any suitable image recognition or comparison software or process to search the database 120 for a matching image or image portion or other parameter. The software or processes used for image recognition may be based on any suitable image analysis algorithm for example, algorithms which match pixel for pixel after one of them is translated, rotated, scaled, distorted and have its brightness and contrast adjusted in a certain specific way, algorithms based on the Neural Networks approach, algorithms based on transformations such as FFT-based correlation algorithms, color histograms, or any other suitable algorithm. In one embodiment, a database search may be performed by a dedicated unit, such as processor 140, for example, a DSP (digital signal processor), an ASIC, an FPGA, a graphics card, an additional processor or another suitable device. Such a dedicated unit may not be needed; for example such processing may be done by a processor operating software.

A match may be made between the image chosen by a health professional and one or more database items, entries or images.. The health professional may receive, for example, the reference image or reference text and/or a description, a report including for example, the diagnosis, related cases, medical information, similar images, similar areas, and similar cases in other stages of the disease or any other information The process of image identification may be performed in different modes, for example, in an automatic mode, in which the system searches the database for a match to any image recorded by in vivo device 40. In for example a semi-automatic mode a health professional may enter or mark for analysis of an image or part of an image (or a number of images) and then may receive as output a diagnosis or match image, or a description for the selected frame(s). In a manual mode a health professional may enter an image or part of an image and request specific information which may be found, for example, in database 120. In one embodiment of the invention, a health professional may get confirmation of a diagnosis or additional information required for better understanding of the patient situation. Other modes or methods for matching may be used.

The search results may be displayed using various suitable methods, for example, a list of results ordered according to image correlation, display of all images correlated to the selected images in the captured image stream or any other suitable method. The search results may include for example a score for the level of correlation that may be displayed graphically as a bar, a ruler, or any other suitable way.

While, preferably, information gathering, storage and processing are performed by certain units, the system and method of the present invention may be practiced with alternate configurations. For example, the components gathering image information need not be contained in a swallowable device but may be contained in any other vehicle suitable for traversing a lumen in a human body, such as an endoscope, stent, catheter, needle etc.

Preferably, data processor storage unit 19 stores a series of images recorded by a device 40. The images the device 40 records as it moves through a patient's GI tract may be combined consecutively or non consecutively to form a moving image. This moving image may be displayed in a window on monitor 18. The moving image may be frozen to view one frame, which may be used for a database search as described, speeded up, or reversed; sections may be skipped; or any other method for viewing an image may be applied to the moving image. While the discussion relates to the case where data from a device 40 is stored for later use, the system and method of the present invention may be used with systems allowing for real time viewing of image data.

In an exemplary embodiment, the moving image is stored as a series of images in the image database 110, and images of pathologies or other references are stored in the pathology database 120. Image database 110 and pathology database 120 may be implemented in a variety of known manners. While, in an exemplary embodiment, image database 110 and pathology database 120 are stored in the same general storage unit, in alternate embodiments image database 110 and pathology database 120 may be stored separately.. Furthermore, the information stored in image database 110 and pathology database 120 may be stored in various combinations; for example, image and pathology information may be stored in the same database.

According to some embodiments, database 120 may include a number of databases, for example, small intestine pathology database, stomach pathology database, annotation database, etc. Some pathology databases may include images and/or additional information of a specific part of the body, for example, specific parts of the GI tract while other databases may include images and/or additional information of certain known diseases, tumors, polyps, lesions, bleeding etc. A health professional may decide to search in more than one database or may decide to start the search in one database and refine the search using another database.

In one embodiment storage unit 19 may include an annotation database, for example, the user may annotate portions of the moving image stream. When used herein, a "portion" of a moving image may include a single still image, a set of still images, or a series of still images, which may be displayed as a moving image. When used herein, "annotation" and its derivatives may indicate any additional item of information or data added to or linked to a moving image or a portion of a moving image, for example, one frame or a number of frames from the moving image. For example, an annotation may include, but is not limited to, a textual, audio, or other note which is associated with a portion of a moving image, a bookmark, tab or label which is associated with a portion of a moving image, a mark on an image or a medical diagnosis or description of the portion.

A bookmark may be, for example, an indication or marker, or an index entry, which indicates to a user a portion of the moving image which is of interest. For example, a user may bookmark a frame which depicts a pathology in a GI tract. An annotation may be useful in a system where a large number of image frames are stored, only a certain number of which are relevant to a diagnosis. The user may store the image sequence and use the annotations to find the relevant portions and to record significant facts about those portions. When wishing to find portions of the moving image which are of interest, the user, or other users, may refer to the bookmarks or annotations.

In an exemplary embodiment, an annotation database may include or refer to sets of images (which may include only one image), a time marking the time elapsed for the image (or the earliest of the set of images), and text. In some embodiments the time may be replaced with another suitable measurement, such as number of frames elapsed, etc. In other embodiments an annotation database may include an image or sets of images that have been marked by a user, for example, marked on a certain part of the image to point out the specific part. Each image may be either an actual image, stored in a known format, or may be a link to an image in the image file. In alternate embodiments, an annotation may include other combinations of information, including, for example, data in a non-textual format. Preferably, the annotations associated with a moving image may be exported from the system or saved as a file, and reports summarizing or otherwise organizing information in the annotations may be generated.

In one embodiment, a health professional or another user may view the image stream received from device 40, may choose an image of interest and may search database(s) 120 for a matching image, and/or relevant information and/or previously annotated images and any other information saved in database 120.

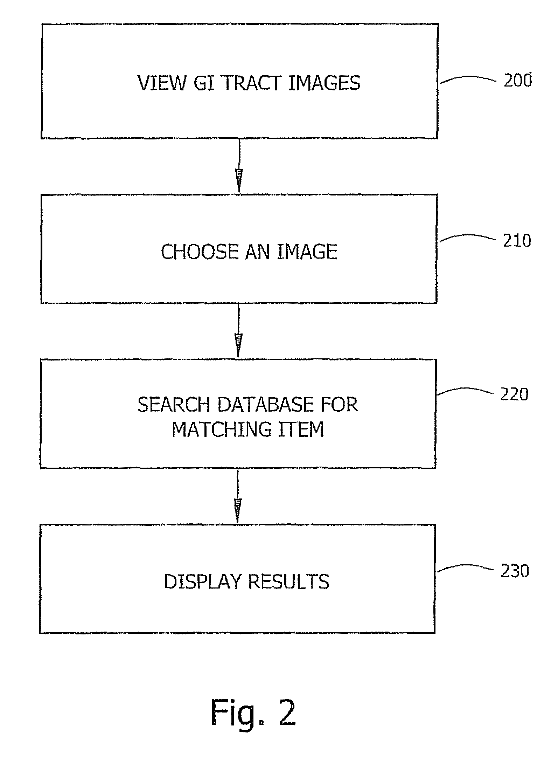

Reference is made to Fig. 2, which depicts a flowchart of an image identification method, according to one embodiment of the present invention. In step 200 the user may view images, for example, GI tract images received from an in vivo device. The images may be stored in a dedicated database or may be viewed in real time while being transferred from an in vivo device located inside a patient's body. The user may view the images on a monitor, which may be a part of a personal computer, a workstation, a PDA etc. In step 210, the user may choose a certain image of interest or a portion of an image. Choosing may be done by operating a user interface, such as a keyboard, a joystick a mouse or any other input device. The image of interest chosen by the user may be displayed in a display screen on a monitor such as the monitor 18. In step 220 the user may perform a search for matching items or entries in one or more databases. In one embodiment, a user may choose to search for a matching image in a pathology database. In another embodiment, the user may search a specific database by entering one or more words of interest or keywords relating to the image of interest. The matching item or entry may include similar images or match an area of image or images highly correlated with selected parts of an image and information matched with a keyword entered by the user Any other search and methods may be used.. The search may use local databases, for example, databases located in a workstation, a personal computer or a remote database, for example, the Internet or a dedicated health database shared with other hospitals or health organizations. In step 230, the results of the search may be displayed on a dedicated display on a monitor or a screen. The search results may include the selected image, a list of matching images, a list of associated texts, articles, publication, medical diagnoses etc. In some embodiments of the invention the search results may be displayed according to image and/or text correlation by a graphic score bar for the level of correlation, ruler or any other way. The results may also be an analysis of an image, a confirmation of a diagnosis, a full report of matched images or the like.

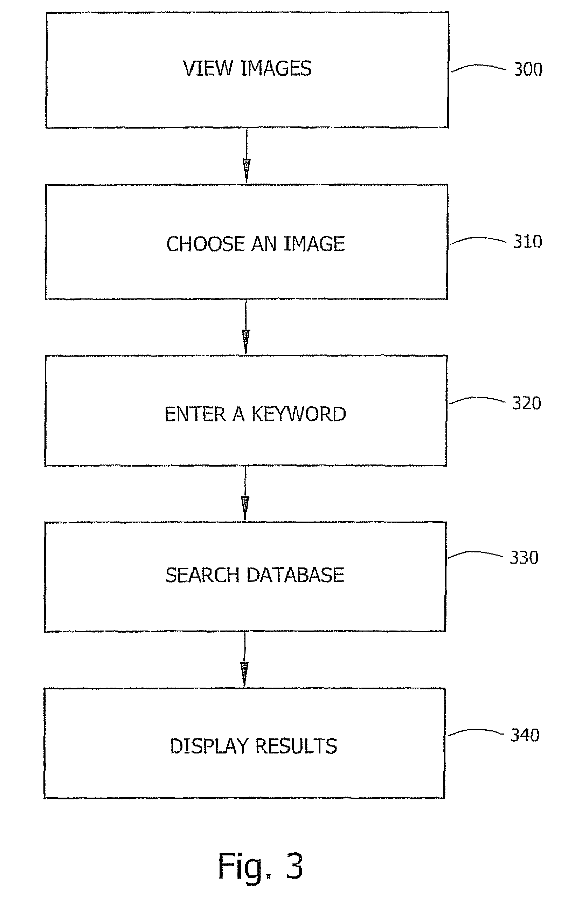

Reference is made to Fig. 3, which depicts a flowchart of an image comparison method, according to one embodiment of the present invention. In step 300 the user may view images, for example, GI tract images received from an in vivo device. The images may be stored in a dedicated database or may be viewed in real time while being transferred from an in vivo device located inside a patient's body. The user may view the images on a monitor, which may be a part of a personal computer, a workstation, a PDA etc. In step 310, the user may choose a certain image of interest and may choose a certain area of interest within the chosen image. Choosing may include by operating a user interface, such as a keyboard, a joystick a mouse or any other input device. The image of interest chosen by the user may be displayed in a display screen on the monitor. In step 320 the user may enter one or more words of interest or keywords relating to the image of interest, which may be relevant for the chosen image and/or for information relates to the chosen image. Any other kinds of suitable keywords may be used.

In step 330 a search may be performed based on the chosen image and/or keywords entered by the user. The search may be performed using one or more databases. For example, a small intestine pathology database, stomach pathology database, annotation database, etc. Some pathology databases may include images and/or additional information of a specific part of the body, for example, specific parts of the GI tract while other databases may include images and/or additional information of certain known diseases, tumors, polyps, lesions, bleeding etc. Some databases may include textual information such as, articles, publications, previous cases analysis and the like.

In some embodiments, the search may give priority to the keyword entered while in other embodiments priority will be given to an image. In some embodiments the priority may be defined according to the user decision. The search may use local databases, for example, databases located in a workstation, a personal computer or a distant database, for example, the Internet or a dedicated health database shared with other hospitals or health organizations. In one embodiment the search may be done only by image correlation and may be refined afterwards by adding keywords or another image, part of an image or any other information.

In step 340, the results of the search may be displayed on a dedicated display on a monitor or a screen. The search results may include the selected image, a list of matching images, a list of associated texts, articles, publication, medical diagnosis etc. In some embodiments of the invention the search results may be displayed according to image and/or text correlation by a graphic score bar for the level of correlation, ruler or using any other suitable method. The results may also include an analysis of an image, a confirmation of a diagnosis, a full report of matched images or the like.

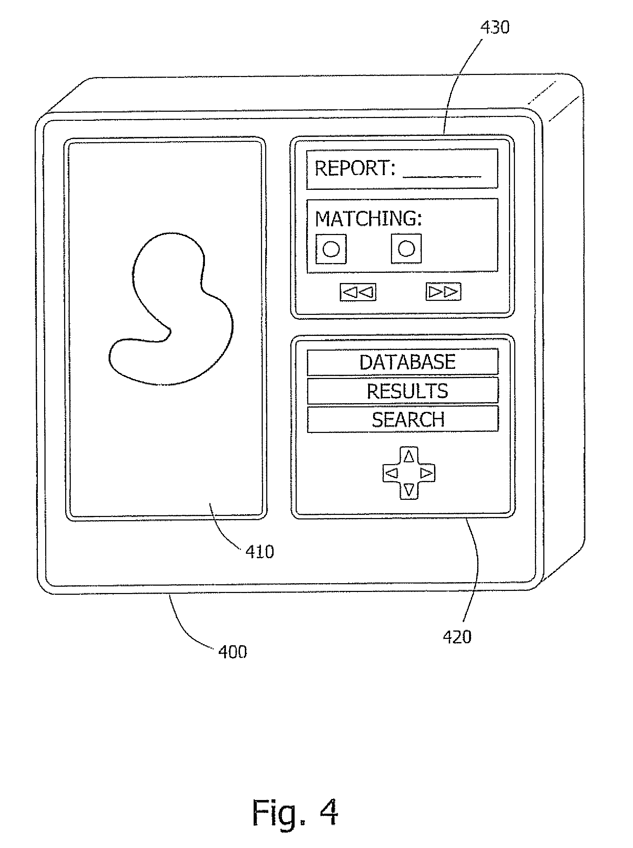

Reference is made to Fig. 4, which shows a display on a monitor according to one embodiment of the present invention. When viewing the moving image, the user may be presented with a set of windows on monitor 400. An image window 410 may provide the image or images received from the in vivo device, or still portions of that image. Such a window may include buttons or other controls which may alter the display of the image; for example, stop, play, pause, capture image, step, fast-forward, rewind, or other controls. Such controls may be activated by, for example, a pointing device such as a mouse or trackball. A timeline window may provide a timeline, an indication of the total time elapsed for the moving image, and may provide other information, such as the total time of the moving image and summaries of annotations. A user command window 420 on monitor 400 may include a set of options arranged, for example, as a menu, bar or buttons. The options may include, for example:
- database selection - the user may choose at least one database out of a databases list.
- search selection - the user may choose which items to search for, for example, images, articles, pathology analysis, etc.
- results selection - the user may choose the search results representation, for example, number of best matches, an analysis of the pathology, a list of search results or any other representation.

### Other suitable options may be used.

The user command window may include a command line for entering the keywords and apply them to the database.

A result window 430 on monitor 400 may show the results of the database search, for example, a list of matching images, a list of associated texts, articles, publication, a medical diagnosis etc. In some embodiments of the invention the search results may be displayed according to image and/or text correlation by a graphic score bar for the level of correlation, ruler or any other suitable method. The results may also be analysis of an image, a confirmation of a diagnosis, a full report of matched images or the like.

The term "window" may relate to areas on a display, not necessarily defined or structured areas. The term "window" may also include a panel including a boarder such as provided by common operating systems.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the scope of the invention.

Throughout the description and claims of this specification, the words "comprise" and "contain" and variations of the words, for example "comprising" and "comprises", means "including but not limited to", and is not intended to (and does not) exclude other moieties, additives, components, integers or steps,

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A method for identifying pathalogy in the GI tract, the method comprising
displaying in vivo images of the GI tract obtained by an in-vivo imaging device;
accepting an indication of an image of interest from the in vivo images; and
searching for an item from a database, the item matching the image of interest.

2. The method of claim 1, wherein the searching is preformed by entering a word relating to the image of interest and/or wherein the item includes an image or text.

3. The method of claim 1 or claim 2, comprising the step of displaying search results.

4. The method of claim 3, comprising displaying simultaneously the image of interest and the search results.

5. The method of claim 3 or claim 4, comprising displaying the image of interest, the search results and an indication of time elapsed for the image of interest.

6. A system for reviewing in vivo images, the system comprising:
an in-vivo imaging device to collect in-vivo images;
a pathology database;
a processor to match an in vivo image with an item in the pathology database; and
a display to display the in-vivo images.

7. The system of claim 6, wherein the pathology database comprises data selected from the group consisting of: images of known pathologies, images of known diseases, images of tumors, images of specific tissues, text information, keywords, descriptions, a complete medical diagnosis, relevant cases, articles or images.

8. The system of claim 6 or claim 7, comprising an image database.

9. The system of claim 8, wherein the image database is configured to be accessed via a network.

10. The system af claim 9, wherein the network is an Internet network.

11. The system of any one of claims 6 to 10, wherein the display is configured to display search results and/or wherein the display comprises a result window and/or wherein an image of interest, a search result and a timeline are displayed simultaneously.

12. The system of claim 11, wherein a graphic score bar is displayed for the level of correlation of the search results and/or wherein the search results are selected from the group consisting of: matching images, associated texts, articles, publication and medical diagnoses.

13. The system of any one of claims 6 to 12, wherein the display comprises a user command window.

14. The system of claim 13, wherein the user command window comprises a set of selection option buttons selected from the group consisting of: database selection , search selection and results selection.
